# EUROPEAN PATENT APPLICATION

(11) **EP 3 741 336 A1**
(43) Date of publication of application: **25.11.2020**
(21) Application number: 20185730.7
(22) Date of filing: 25.10.2017
(51) Int. Cl.: A61F 13/00, A61F 13/02

(54) **HYBRID DRAPE**

(30) Priority: 26.10.2016 US 201662413131 P
(62) Divisional of application: 17794612.6
(71) Applicant: 3M Innovative Properties Company, St. Paul, MN 55133-3427 (US)
(72) Inventor: LOCKE, Christopher, Brian, San Antonio, TX Texas 78265 (US); ROBINSON, Timothy, Mark, San Antonio, TX Texas 78265 (US)
(74) Representative: Simmons & Simmons

(57) **Abstract**

A sealing member for providing a seal over a tissue site and a method of making the same is described. The sealing member includes a film layer; a first adhesive coupled to and coextensive with the film layer; and a second adhesive coupled to and coextensive with the first adhesive. A plurality of apertures extend through the second adhesive. Each aperture defines an interior surface between a first side and a second side of the second adhesive. The interior surface intersects the first side to form a first edge and intersects the second side to form a second edge, the interior surface comprising a chamfer. The first adhesive is configured to extend at least partially through the plurality of apertures.

## Description

### RELATED APPLICATIONS

This application claims the benefit, under 35 USC 119(e), of the filing of U.S. Provisional Patent Application No. 62/413,131, entitled "Hybrid Drape and Method of Manufacturing a Hybrid Drape," filed October 26, 2016, which is incorporated herein by reference for all purposes.

### TECHNICAL FIELD

The invention set forth in the appended claims relates generally to tissue treatment systems and more particularly, but without limitation, to sealing systems and methods to manufacture a hybrid drape.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

There is also widespread acceptance that cleansing a tissue site can be highly beneficial for new tissue growth. For example, a wound can be washed out with a stream of liquid solution, or a cavity can be washed out using a liquid solution for therapeutic purposes. These practices are commonly referred to as "irrigation" and "lavage" respectively. "Instillation" is another practice that generally refers to a process of slowly introducing fluid to a tissue site and leaving the fluid for a prescribed period of time before removing the fluid. For example, instillation of topical treatment solutions over a wound bed can be combined with negative-pressure therapy to further promote wound healing by loosening soluble contaminants in a wound bed and removing infectious material. As a result, soluble bacterial burden can be decreased, contaminants removed, and the wound cleansed.

While the clinical benefits of negative-pressure therapy and/or instillation therapy are widely known, improvements to therapy systems, components, and processes may benefit healthcare providers and patients.

### BRIEF SUMMARY

New and useful systems, apparatuses, and methods for providing a sealed space in a negative-pressure therapy environment are set forth in the appended claims. Illustrative embodiments are also provided to enable a person skilled in the art to make and use the claimed subject matter.

In some embodiments, a sealing member for providing a seal over a tissue site is described. The sealing member can include a film layer; a first adhesive coupled to and coextensive with the film layer; and a second adhesive coupled to and coextensive with the first adhesive. A plurality of apertures can extend through the second adhesive. Each aperture defines an interior surface between a first side and a second side of the second adhesive. The interior surface can intersect the first side to form a first edge and intersect the second side to form a second edge, the interior surface comprising a chamfer. The first adhesive is configured to extend at least partially through the plurality of apertures.

More generally, a cover for providing a seal over a tissue site is described. The cover can include a polyurethane layer; an acrylic adhesive coupled to and coextensive with the polyurethane layer; and a silicone adhesive coupled to and coextensive with the acrylic adhesive. A plurality of openings can extend through the silicone adhesive. Each opening may have a first diameter opposite the acrylic adhesive and a second diameter adjacent the acrylic adhesive. The acrylic adhesive can be configured to extend at least partially through the plurality of openings.

Alternatively, other example embodiments may describe a drape for providing a seal over a tissue site. The drape can include a sealing layer having a plurality of apertures extending through the sealing layer. The drape can also include a plurality of tapered surfaces formed by the plurality of apertures in the sealing layer, a bonding layer coupled to the sealing layer; and a barrier layer coupled to the bonding layer on a side of the bonding layer opposite the sealing layer.

A method of manufacturing a sealing member is also described. In the method, a carrier layer is provided and coated with a first adhesive. The first adhesive can be cured to form a first adhesive layer. Energy can be applied to a plurality of circular areas of the first adhesive layer to transition the circular areas from a cured state to a precursor state. The first adhesive layer can be compressed to form a plurality of depressions registered with the plurality of circular areas. Energy can be applied to the plurality of circular areas of the first adhesive layer to transition the circular areas from the precursor state to the cured state. At least a portion of each depression of the first adhesive layer can be removed to form an aperture registered with each depression. A second adhesive can be coupled to a film layer to form a second adhesive layer, and the first adhesive layer can be coupled to the second adhesive layer. The carrier layer can be removed.

Another method of manufacturing a cover is described. A first adhesive layer can be supplied on carrier layer, the first adhesive layer comprising a silicone adhesive in a cured state. Energy can be supplied to the first adhesive layer to transition the silicone adhesive from a cured state to a precursor state. A plurality of depressions can be formed in the silicone adhesive of the first adhesive layer. Energy can be supplied to the first adhesive layer to transition the silicone adhesive from the precursor state to the cured state. A portion of the silicone adhesive can be removed at each depression of the first adhesive layer to form a plurality of openings registered with the plurality of depressions. A second adhesive layer can be coupled to a film layer, and the first adhesive layer can be coupled to the second adhesive layer. The carrier layer can be removed.

Objectives, advantages, and a preferred mode of making and using the claimed subject matter may be understood best by reference to the accompanying drawings in conjunction with the following detailed description of illustrative embodiments.

### BRIEF DESCRIPTION OF THE DRAWINGS

Figure 1 is a sectional view, with a portion shown in elevation, of an example embodiment of a therapy system that can provide a sealed therapeutic environment in accordance with this specification;
Figure 2 is a sectional exploded view of a portion of a cover, illustrating additional details that may be associated with an example embodiment of the therapy system of Figure 1;
Figure 3 is a sectional view of the portion of the cover of Figure 2, illustrating additional details that may be associated with an example embodiment of the therapy system of Figure 1;
Figure 4 is a bottom plan view of the cover, illustrating additional details that may be associated with an example embodiment of the therapy system of Figure 1;
Figure 5 is a detail top plan view of an adhesive layer of the cover of Figure 4, illustrating additional details that may be associated with some embodiments;
Figure 6 is a sectional view of a portion of another cover that may be used with the therapy system of Figure 1;
Figure 7 is a sectional view of a portion of another cover that may be used with the therapy system of Figure 1;
Figure 8 is a sectional view of a portion of another cover that may be used with the therapy system of Figure 1;
Figures 9-15 are schematic sectional views of a process for manufacturing the cover of Figure 1;
Figure 16 is a schematic perspective view of another manufacturing process for producing the cover of Figure 1;
Figure 17 is a schematic sectional view of a portion of the manufacturing process of Figure 16 taken along line 17-17; and
Figure 18 is a schematic sectional view of a portion of the manufacturing process of Figure 17.

### DESCRIPTION OF EXAMPLE EMBODIMENTS

The following description of example embodiments provides information that enables a person skilled in the art to make and use the subject matter set forth in the appended claims, but may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

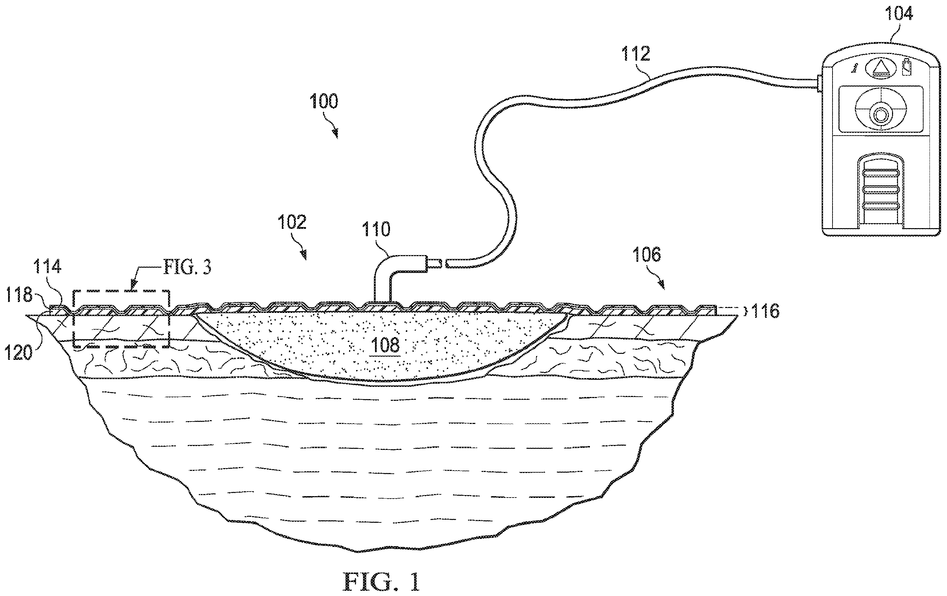

Figure 1 is a sectional view with a portion shown in elevation of an example embodiment of a therapy system 100 that can provide negative-pressure therapy and/or instillation of topical treatment solutions in accordance with this specification. The term "tissue site" in this context broadly refers to a wound, defect, or other treatment target located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a negative-pressure supply and may include or be configured to be coupled to a distribution component, such as a dressing. In general, a distribution component may refer to any complementary or ancillary component configured to be fluidly coupled to a negative-pressure supply in a fluid path between the negative-pressure supply and a tissue site. A distribution component is preferably detachable, and may be disposable, reusable, or recyclable. For example, a dressing 102 may be fluidly coupled to a negative-pressure source 104, as illustrated in Figure 1. A dressing may include a cover, a tissue interface, or both in some embodiments. The dressing 102, for example, may include a cover 106 and a tissue interface 108. The cover 106 can include an elastomeric film 114 and an attachment device 116. In some embodiments, the attachment device 116 includes a first adhesive layer 118 and a second adhesive layer 120. A regulator or a controller may also be coupled to the negative-pressure source 104.

In some embodiments, a dressing interface, such as a dressing interface 110, may facilitate coupling the negative-pressure source 104 to the dressing 102. For example, the dressing interface 110 may be a T.R.A.C.® Pad or Sensa T.R.A.C.® Pad available from KCI of San Antonio, Texas. The therapy system 100 may optionally include a fluid container coupled to the dressing 102 and to the negative-pressure source 104.

The therapy system 100 may also include a source of instillation solution. For example, a solution source may be fluidly coupled to the dressing 102. The solution source may be fluidly coupled to a positive-pressure source in some embodiments, or may be fluidly coupled to the negative-pressure source 104. A regulator may also be fluidly coupled to the solution source and the dressing 102. In some embodiments, an instillation regulator may also be fluidly coupled to the negative-pressure source 104 through the dressing 102.

Additionally, the therapy system 100 may include sensors to measure operating parameters and provide feedback signals to a controller indicative of the operating parameters. The therapy system 100 may include a pressure sensor, an electric sensor, or both, coupled to a controller. A pressure sensor may also be coupled or configured to be coupled to a distribution component and to the negative-pressure source 104.

Components may be fluidly coupled to each other to provide a path for transferring fluids (i.e., liquid and/or gas) between the components. For example, components may be fluidly coupled through a fluid conductor, such as a tube 112. A "tube," as used herein, broadly includes a tube, pipe, hose, conduit, or other structure with one or more lumina adapted to convey a fluid between two ends. Typically, a tube is an elongated, cylindrical structure with some flexibility, but the geometry and rigidity may vary. In some embodiments, components may also be coupled by virtue of physical proximity, being integral to a single structure, or being formed from the same piece of material. Moreover, some fluid conductors may be molded into or otherwise integrally combined with other components. Coupling may also include mechanical, thermal, electrical, or chemical coupling (such as a chemical bond) in some contexts. For example, the tube 112 may mechanically and fluidly couple the dressing 102 to the negative-pressure source 104.

In general, components of the therapy system 100 may be coupled directly or indirectly. For example, the negative-pressure source 104 may be directly coupled to the dressing interface 110. The negative-pressure source 104 may be indirectly coupled to the dressing 102 through the dressing interface 110.

The fluid mechanics of using a negative-pressure source to reduce pressure in another component or location, such as within a sealed therapeutic environment, can be mathematically complex. However, the basic principles of fluid mechanics applicable to negative-pressure therapy and instillation are generally well-known to those skilled in the art, and the process of reducing pressure may be described illustratively herein as "delivering," "distributing," or "generating" negative pressure, for example.

In general, exudates and other fluids flow toward lower pressure along a fluid path. Thus, the term "downstream" typically implies a position in a fluid path relatively closer to a source of negative pressure or further away from a source of positive pressure. Conversely, the term "upstream" implies a position relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications (such as by substituting a positive-pressure source for a negative-pressure source) and this descriptive convention should not be construed as a limiting convention.

"Negative pressure" generally refers to a pressure less than a local ambient pressure, such as the ambient pressure in a local environment external to a sealed therapeutic environment provided by the dressing 102. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. Similarly, references to increases in negative pressure typically refer to a decrease in absolute pressure, while decreases in negative pressure typically refer to an increase in absolute pressure. While the amount and nature of negative pressure applied to a tissue site may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -75 mm Hg (-9.9 kPa) and -300 mm Hg (-39.9 kPa).

A negative-pressure supply, such as the negative-pressure source 104, may be a reservoir of air at a negative pressure, or may be a manual or electrically-powered device that can reduce the pressure in a sealed volume, such as a vacuum pump, a suction pump, a wall suction port available at many healthcare facilities, or a micro-pump, for example. A negative-pressure supply may be housed within or used in conjunction with other components, such as sensors, processing units, alarm indicators, memory, databases, software, display devices, or user interfaces that further facilitate therapy. For example, in some embodiments, the negative-pressure source 104 may be combined with a controller and other components into a therapy unit. A negative-pressure supply may also have one or more supply ports configured to facilitate coupling and de-coupling the negative-pressure supply to one or more distribution components.

A controller may be a microprocessor or computer programmed to operate one or more components of the therapy system 100, such as the negative-pressure source 104. In some embodiments, for example, a controller may be a microcontroller, which generally comprises an integrated circuit containing a processor core and a memory programmed to directly or indirectly control one or more operating parameters of the therapy system 100. Operating parameters may include the power applied to the negative-pressure source 104, the pressure generated by the negative-pressure source 104, or the pressure distributed to the tissue interface 108, for example. A controller is also preferably configured to receive one or more input signals, such as a feedback signal, and programmed to modify one or more operating parameters based on the input signals.

Sensors, such as a pressure sensor or an electric sensor, are generally known in the art as any apparatus operable to detect or measure a physical phenomenon or property, and generally provide a signal indicative of the phenomenon or property that is detected or measured. For example, a pressure sensor and an electric sensor may be configured to measure one or more operating parameters of the therapy system 100. In some embodiments, a pressure sensor may be a transducer configured to measure pressure in a pneumatic pathway and convert the measurement to a signal indicative of the pressure measured. In some embodiments, for example, a pressure sensor may be a piezoresistive strain gauge. An electric sensor may optionally measure operating parameters of the negative-pressure source 104, such as the voltage or current, in some embodiments. Preferably, the signals from a pressure sensor and an electric sensor are suitable as an input signal to a controller, but some signal conditioning may be appropriate in some embodiments. For example, the signal may need to be filtered or amplified before it can be processed by the controller. Typically, the signal is an electrical signal, but may be represented in other forms, such as an optical signal.

In some embodiments, the negative-pressure source 104 can include a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

In some embodiments, the negative-pressure source 104 may be a therapy unit having a solution source. The solution source may also be representative of a container, canister, pouch, bag, or other storage component, which can provide a solution for instillation therapy. Compositions of solutions may vary according to a prescribed therapy, but examples of solutions that may be suitable for some prescriptions include hypochlorite-based solutions, silver nitrate (0.5%), sulfur-based solutions, biguanides, cationic solutions, and isotonic solutions.

The tissue interface 108 can be generally adapted to contact a tissue site. The tissue interface 108 may be partially or fully in contact with the tissue site. If the tissue site is a wound, for example, the tissue interface 108 may partially or completely fill the wound, or may be placed over the wound. The tissue interface 108 may take many forms, and may have many sizes, shapes, or thicknesses depending on a variety of factors, such as the type of treatment being implemented or the nature and size of a tissue site. For example, the size and shape of the tissue interface 108 may be adapted to the contours of deep and irregular shaped tissue sites. Moreover, any or all of the surfaces of the tissue interface 108 may have projections or an uneven, course, or jagged profile that can induce strains and stresses on a tissue site, which can promote granulation at the tissue site.

In some embodiments, the tissue interface 108 may be a manifold. A "manifold" in this context generally includes any substance or structure providing a plurality of pathways adapted to collect or distribute fluid across a tissue site under pressure. For example, a manifold may be adapted to receive negative pressure from a source and distribute negative pressure through multiple apertures across a tissue site, which may have the effect of collecting fluid from across a tissue site and drawing the fluid toward the source. In some embodiments, the fluid path may be reversed or a secondary fluid path may be provided to facilitate delivering fluid across a tissue site.

In some illustrative embodiments, the pathways of a manifold may be interconnected to improve distribution or collection of fluids across a tissue site. In some illustrative embodiments, a manifold may be a porous foam material having interconnected cells or pores. For example, cellular foam, open-cell foam, reticulated foam, porous tissue collections, and other porous material such as gauze or felted mat generally include pores, edges, and/or walls adapted to form interconnected fluid channels. Liquids, gels, and other foams may also include or be cured to include apertures and fluid pathways. In some embodiments, a manifold may additionally or alternatively comprise projections that form interconnected fluid pathways. For example, a manifold may be molded to provide surface projections that define interconnected fluid pathways.

The average pore size of a foam may vary according to needs of a prescribed therapy. For example, in some embodiments, the tissue interface 108 may be a foam having pore sizes in a range of about 400 to about 600 microns. The tensile strength of the tissue interface 108 may also vary according to needs of a prescribed therapy. For example, the tensile strength of a foam may be increased for instillation of topical treatment solutions. In one non-limiting example, the tissue interface 108 may be an open-cell, reticulated polyurethane foam such as GranuFoam® dressing or VeraFlo® foam, both available from Kinetic Concepts, Inc. of San Antonio, Texas.

The tissue interface 108 may be either hydrophobic or hydrophilic. In an example in which the tissue interface 108 may be hydrophilic, the tissue interface 108 may also wick fluid away from a tissue site, while continuing to distribute negative pressure to the tissue site. The wicking properties of the tissue interface 108 may draw fluid away from a tissue site by capillary flow or other wicking mechanisms. An example of a hydrophilic foam is a polyvinyl alcohol, open-cell foam such as V.A.C. WhiteFoam® dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity.

The tissue interface 108 may further promote granulation at a tissue site if pressure within the sealed therapeutic environment is reduced. For example, any or all of the surfaces of the tissue interface 108 may have an uneven, coarse, or jagged profile that can induce microstrains and stresses at a tissue site if negative pressure is applied through the tissue interface 108.

In some embodiments, the tissue interface 108 may be constructed from bioresorbable materials. Suitable bioresorbable materials may include, without limitation, a polymeric blend of polylactic acid (PLA) and polyglycolic acid (PGA). The polymeric blend may also include without limitation polycarbonates, polyfumarates, and capralactones. The tissue interface 108 may further serve as a scaffold for new cell-growth, or a scaffold material may be used in conjunction with the tissue interface 108 to promote cell-growth. A scaffold is generally a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials.

The cover 106 may provide a bacterial barrier and protection from physical trauma. The cover 106 may also be constructed from a material that can reduce evaporative losses. The cover 106 can also be constructed from a material or materials that provide a fluid seal between two components or two environments, such as between a therapeutic environment and a local external environment.

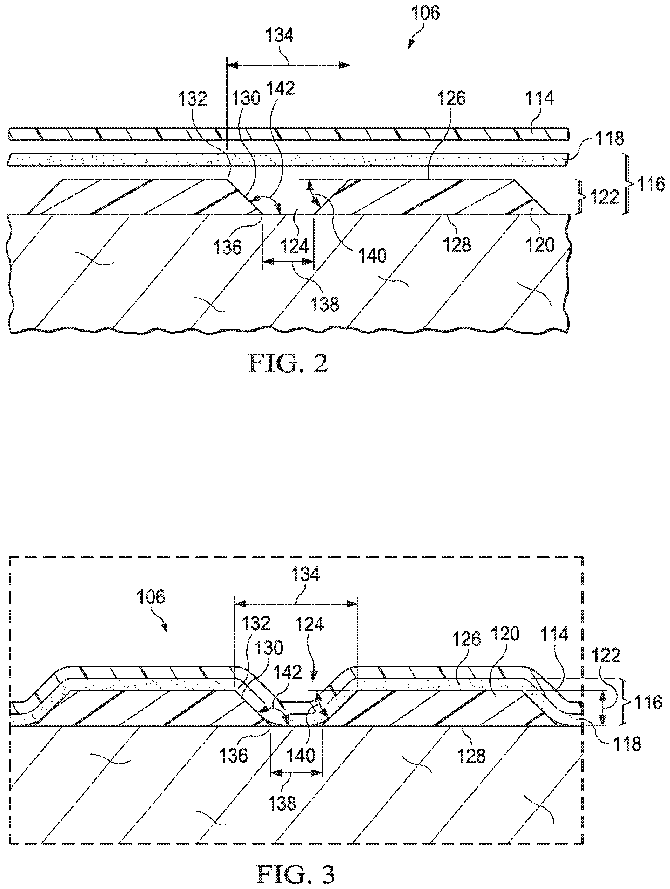

Figure 2 is a sectional exploded view of a portion of the cover 106, illustrating additional details that may be associated with some embodiments. The elastomeric film 114 may be, for example, a film layer formed from an elastomeric film, polyurethane, or membrane that can provide a seal adequate to maintain a negative pressure at a tissue site for a given negative-pressure source. The elastomeric film 114 may have a high moisture-vapor transmission rate (MVTR) in some applications. For example, the MVTR may be at least about 300 g/m^2 per twenty-four hours. In some embodiments, the elastomeric film 114 may be a polymer drape, such as a polyurethane film, that is permeable to water vapor but impermeable to liquid. Such drapes typically have a thickness in the range of about 25 microns to about 50 microns. For permeable materials, the permeability generally should be low enough that a desired negative pressure may be maintained.

In some embodiments, the elastomeric film 114 may include a plurality of perforations extending through the elastomeric film 114. The plurality of perforations may be covered by the attachment device 116. In some embodiments, the plurality of perforations may permit a switching solution, such as isopropyl alcohol to be applied to the attachment device 116 through the elastomeric film 114. The application of isopropyl alcohol to the attachment device 116 may cause the attachment device to decrease in bond strength, aiding in release of the cover 106.

The attachment device 116 may be used to attach the cover 106 to an attachment surface, such as undamaged epidermis, a gasket, or another cover. The attachment device 116 may take many forms. For example, the attachment device 116 may be a medically-acceptable, pressure-sensitive adhesive that extends about a periphery, a portion, or an entire sealing member. In some embodiments, for example, some or all of the cover 106 may be coated with an acrylic adhesive having a coating weight between about 25 grams per square meter and about 45 grams per square meter (gsm). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

In some embodiments, the attachment device 116 includes a first adhesive layer 118 and a second adhesive layer 120. Adjacent to the elastomeric film 114 is the first adhesive layer 118. The first adhesive layer 118 has a first side in contact with the elastomeric film 114 and a second side facing away from the elastomeric film 114. The first adhesive layer 118 may be any medically-acceptable, pressure-sensitive adhesive. For example, the first adhesive layer 118 may comprise an acrylic adhesive, rubber adhesive, high-tack silicone adhesive, polyurethane, or other substance. In an illustrative example, the first adhesive layer 118 comprises an acrylic adhesive with a coating weight of about 15 grams/m² (gsm) to about 70 grams/m² (gsm). In some embodiments, the first adhesive layer 118 may have a bond strength of about 9.5 N/25.4 mm measured on a stainless steel substrate at 23° C at 50% relative humidity based on the American Society for Testing and Materials ("ASTM") standard ASTM D3330. Suitable bond strengths for the first adhesive layer 118 may range from about 15% less than 9.5 N/25.4 mm to about 15% greater than 9.5 N/25.4 mm. In some embodiments, the bond strength of the first adhesive layer 118 may be about 20 N/25.4 mm.

In some embodiments, the first adhesive layer 118 may be a continuous layer of material. In other embodiments, the first adhesive layer 118 may have apertures (not shown). The apertures may be formed after application of the first adhesive layer 118 or may be formed by coating the first adhesive layer 118 in patterns on a carrier layer, e.g., a side of the elastomeric film 114. The apertures in the first adhesive layer 118 may be sized to help control the resultant bond between epidermis surrounding a tissue site and the first adhesive layer 118. The apertures may also be sized to enhance the MVTR of the cover 106.

The second adhesive layer 120 has a first surface 126 in contact with the first adhesive layer 118 and a second surface 128 opposite the first surface 126. The first surface 126 may be oriented toward the first adhesive layer 118. In some embodiments, the first surface 126 may be adjacent to and facing the first adhesive layer 118. The second surface 128 may face away from the first adhesive layer 118. In some embodiments, the second surface 128 may be configured to contact epidermis adjacent a tissue site during use of the cover 106.

The second adhesive layer 120 is a soft material that provides a good seal with the epidermis 103. The second adhesive layer 120 may comprise a silicone gel (or soft silicone), hydrocolloid, hydrogel, polyurethane gel, polyolefin gel, hydrogenated styrenic copolymer gels, or foamed gels with compositions as listed, or soft closed cell foams (polyurethanes, polyolefins) coated with an adhesive (e.g., about 30 gsm to about 70 gsm acrylic), polyurethane, polyolefin, or hydrogenated styrenic copolymers. In some embodiments, the second adhesive layer 120 may include a scrim layer disposed within the second adhesive layer 120. The scrim layer may support the second adhesive layer 120 during the manufacture of the cover 106. The second adhesive layer 120 may also include a carboxymethyl cellulose (CMC) disposed within the second adhesive layer 120. The CMC may aid moisture management. The second adhesive layer 120 may have a thickness 122 that is typically in the range of about 500 microns (µm) to about 1000 microns (µm). The second adhesive layer 120 may have stiffness between about 5 Shore OO and about 80 Shore OO. The second adhesive layer 120 may be hydrophobic or hydrophilic. In some embodiments, the second adhesive layer 120 may have a bond strength of about 0.6 N/25.5mm measured on a stainless steel substrate at 23° C at 50% relative humidity based on the ASTM D3330. Suitable bond strengths for the second adhesive layer 120 may range from about 15% less than 0.6 N/22 mm to about 15% greater than 0.6 N/22 mm.

The second adhesive layer 120 is formed with a plurality of apertures 124. The apertures 124 may be openings having numerous shapes, for example, circles, squares, stars, ovals, polygons, slits, complex curves, rectilinear shapes, triangles, or other shapes. Each aperture 124 of the plurality of apertures 124 has an effective diameter. An effective diameter is the diameter of a circle having the same area as a non-circular opening. The average effective diameter of each aperture 124 is typically in the range of about 4 mm to about 50 mm. The plurality of apertures 124 may have a uniform pattern or may be randomly distributed in the second adhesive layer 120.

The provision of negative-pressure therapy with negative-pressure therapy systems, such as the therapy system 100, is increasingly being performed with smaller therapy devices that use batteries to provide power to a pump rather than a connection to an electrical outlet. Use of batteries decreases the total power supply available to a therapy device. As a result, power drains that would be considered negligible in a device powered through an electrical outlet connection may significantly reduce the ability of the therapy device to provide therapy. A power drain refers to operation of the therapy device that requires use of electrical power, for example, operation of a pump to generate reduced pressure. Power drains may be caused by low-level dressing leaks, for example. A low-level dressing leak can drain power from a battery of a therapy device by repeatedly triggering operation of the therapy device to maintain the necessary reduced pressure at the tissue site. These power drains shorten the useful life of the therapy device before disposal of the therapy device, recharge of the battery, or battery replacement is required. Leak detection techniques may help to identify some leaks that may be then sealed by the user; however, low level leaks will challenge the most sensitive leak detection systems and may often go undetected.

Low level dressing leaks may occur between the cover and the epidermis surrounding a tissue site when the cover fails to completely seal to the epidermis. Covers are a balance between the strength of the adhesive required to enable the cover to seal against leaks and the pain which may result when the cover is removed. A bonding adhesive may be better for sealing, but the adhesive strength would cause significantly more discomfort upon cover removal. In addition, removing a cover with a bonding adhesive may cause significant damage to patients having delicate or damaged skin.

A cover that has a sealing adhesive can fill gaps between the drape and the epidermis to limit leaks and can be easy to remove with low discomfort to the patient. Various sealing, gap-filling adhesives, such as silicone, hydrocolloids, and hydrogels, have been tried but each has drawbacks. For example, hydrogel adhesives are usually low tack and prone to swelling, creep, and mobility when used with fluid systems. In another example, silicone adhesives can fill gaps and seal, but are not breathable and may lose the necessary mechanical bonding strength as the silicone adhesives interact with moisture during use. To counter these problems, silicone adhesives often require additional materials to secure the silicone adhesive to the patient. For example, a low leak cover may be formed from two adhesive layers: a thick sealing adhesive, perhaps in the shape of a gasket or ring, and a thinner bonding adhesive layer used to keep the sealing adhesive in place. The thinner bonding adhesive may be applied as cover strips, or combined with the thicker sealing adhesive as an outer border. Low-leak covers constructed in this way can be more complex than a cover using a single adhesive, increasing the complexity of manipulation and operation.

Covers having a hybrid configuration may overcome some of these problems. For example, a hybrid cover having a film layer, a bonding adhesive adjacent the film layer, a sealing adhesive adjacent the bonding adhesive, and a plurality of apertures exposing the bonding adhesive through the sealing adhesive may resolve problems of covers using a sealing adhesive or a bonding adhesive alone. However, hybrid covers compromise between the sealing area of a cover and the adhesion area of a cover. The sealing area of a cover may be the fractional surface area of the cover comprising a sealing adhesive that can seal to a tissue site. The adhesion area of a cover is the fractional area of the cover comprising a bonding adhesive that can contact and adhere to a tissue site. In some covers, the ratio between the sealing area and the adhesion area of a cover can be between 1:3 and 1:1. For example, some hybrid covers may have approximately 40% of the surface area of the cover comprising a silicone adhesive and approximately 60% of the surface area of the cover comprising an acrylic adhesive. Other covers may have approximately 50% of the surface area of the cover comprising a sealing adhesive and approximately 50% of the surface area of the cover comprising a bonding adhesive. To improve adhesion of a cover more of the bonding adhesive should be capable of adhering to a tissue site. However, increasing the adhesion area may lead to reduction of the sealing area. Reduction of the sealing area can lead to more trauma for a patient and a higher chance of leaks between the cover and the epidermis. Some covers attempt to address this issue by increasing the bond strength of the bonding adhesive. However, bond strengths above 20.3 N/ 25.4 mm can cause significant tissue damage during removal of the cover. Consequently, increasing the bond strength of the bonding adhesive beyond 20.3 N/ 25.4 mm does not provide a suitable solution.

Some hybrid covers hold the bonding adhesive away from the tissue upon application of the cover. This occurs because of the thickness of the sealing adhesive, which can cause a gap between the bonding adhesive and the tissue. The cover can be pressed to force the bonding adhesive through the gaps. However, the thickness of the sealing adhesive may prevent the bonding adhesive from completely filling the gap, creating an annulus between the bonding adhesive that contacts the tissue and the sealing adhesive that contacts the tissue. The annulus can decrease the adhesion area. For example, an area of the bonding adhesive in contact with the tissue through a 10 mm diameter aperture in the sealing adhesive may only have an effective diameter between about 8 mm and 9 mm.

The therapy system 100 can overcome these problems and others by providing the cover 106 having the elastomeric film 114 and the attachment device 116, which comprises the first adhesive layer 118 and the second adhesive layer 120. The first adhesive layer 118 may have a higher bond strength than the second adhesive layer 120 so that the first adhesive layer 118 can hold the cover 106 to epidermis surrounding a tissue site. In some embodiments, the apertures 124 may be contoured. The contour of the apertures 124 can increase the area of the first adhesive layer 118 in contact with the epidermis. By increasing the area of the first adhesive layer 118 in contact with the epidermis, i.e., the adhesion area, the cover 106 can better adhere to the tissue site and surrounding epidermis.

Each aperture 124 may form a third surface 130 extending from the first surface 126 to the second surface 128. In some embodiments, the third surface 130 may be an interior surface that is cylindrical, conical, frusto-conical, or may comprise a plurality of surfaces joined to form the aperture 124. The third surface 130 may intersect the first surface 126 to form a first edge 132 having a first diameter 134. The third surface 130 may also form an angle 140 with the first surface 126. The third surface 130 may intersect the second surface 128 to form a second edge 136 having a second diameter 138. The third surface 130 may form an angle 142 with the second surface 128. Generally, the angle 140 and the angle 142 are interior angles between the parallel surfaces of the first surface 126 and the second surface 128 so that the sum of the angle 140 and the angle 142 may be about 180 degrees. The angle 140 may be between about 20 degrees and about 60 degrees and preferably between about 30 degrees and about 45 degrees. The angle 142 may be between about 160 and about 120 degrees and preferably between about 150 degrees and about 135 degrees. The aperture 124 is chamfered to form the third surface 130. For example, the first diameter 134 can be larger than the second diameter 138 so that the third surface 130 is a tapered surface or a chamfer between the first edge 132 and the second edge 136.

The first diameter 134 may be an effective diameter, so that the first edge 132 forms a non-circular shape. Similarly, the second diameter 138 may be an effective diameter so that the second edge 136 forms a non-circular shape. In some embodiments, the effective diameter of the aperture 124 may be the second diameter 138. In other embodiments, the effective diameter of the aperture 124 may be the first diameter 134 or an average of the first diameter 134 and the second diameter 138.

Figure 3 is a sectional view of a portion of the cover 106 illustrating additional details that may be associated with the cover 106 in an assembled state. The first adhesive layer 118 and the elastomeric film 114 may be disposed in the plurality of apertures 124. In some embodiments, a surface of the first adhesive layer 118 may be proximate to the second surface 128 of the second adhesive layer 120. If the surface of the first adhesive layer 118 is proximate to the second surface 128 of the second adhesive layer 120, the first adhesive layer 118 and the elastomeric film 114 that is coupled to the first adhesive layer 118 may substantially fill the plurality of apertures 124. The first adhesive layer 118 may be in contact with and coupled to the third surface 130.

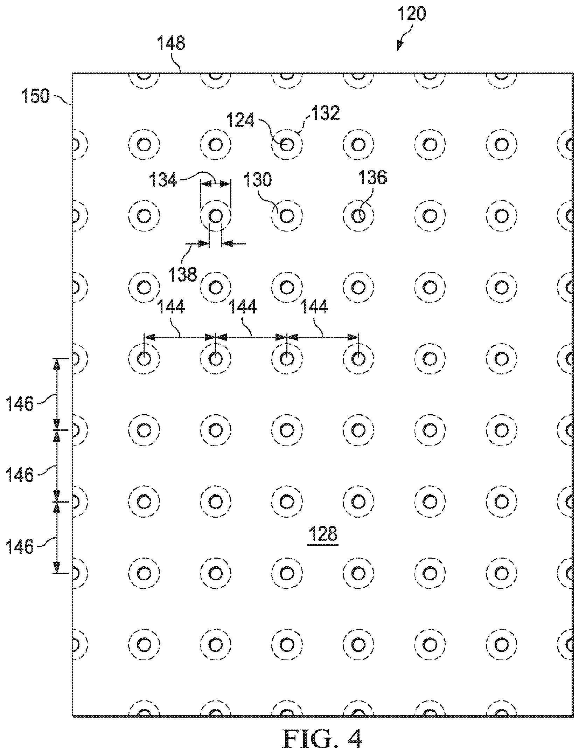

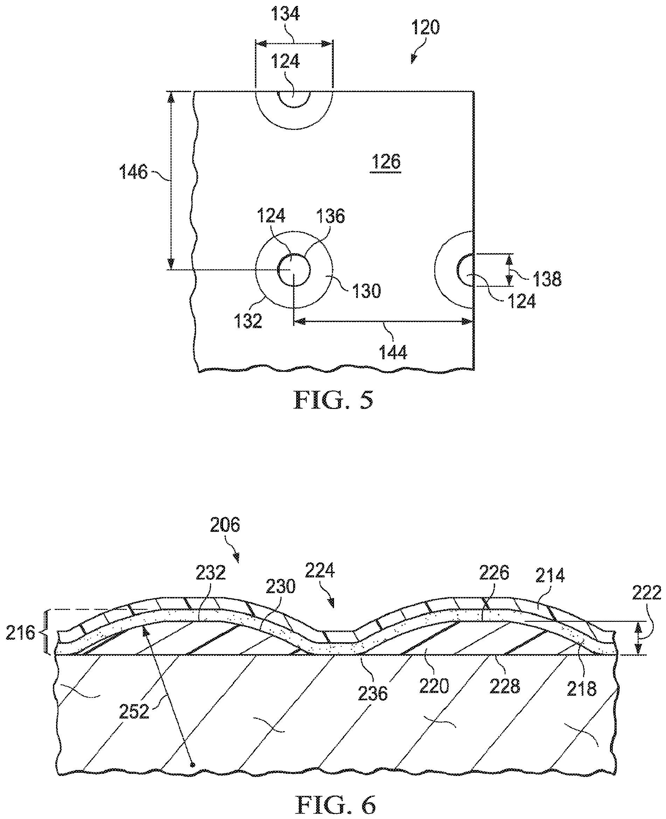

Figure 4 is a bottom plan view of the second adhesive layer 120, and Figure 5 is a top plan view of a portion of the second adhesive layer 120 illustrating additional details that may be associated with some embodiments. The apertures 124 may have a pitch 144 between adjacent apertures 124 in a first direction. Pitch describes a spacing between objects having translational symmetry. The apertures 124 may have a pitch 146 between adjacent apertures 124 in a second direction. In some embodiments, the first direction and the second direction may be orthogonal. In other embodiments, the first direction and the second direction may not be orthogonal. The pitch 144 may be parallel to a first edge 148 of the second adhesive layer 120, and the pitch 146 may be parallel to a second edge 150 of the second adhesive layer 120. In other embodiments, the pitch 144 and the pitch 146 between adjacent apertures 124 may not be regularly repeating, may not be parallel to the first edge 148 and the second edge 150, and may not be continuous across the second adhesive layer 120.

Figure 6 is a sectional view of a portion of another cover 206 illustrating additional details that may be associated with some embodiments. The cover 206 may include an elastomeric film 214 and an attachment device 216. The elastomeric film 214 and the attachment device 216 may be similar to and operate as described above with respect to the elastomeric film 114 and the attachment device 116. The attachment device 216 may include a first adhesive layer 218 and a second adhesive layer 220. The first adhesive layer 218 and the second adhesive layer 220 may be similar to and operate as described above with respect to the first adhesive layer 118 and the second adhesive layer 120. The second adhesive layer 220 may include a plurality of apertures 224 that may be similar to and operate as described above with respect to the plurality of apertures 124. The second adhesive layer 220 may have a first surface 226, a second surface 228, and a thickness 222 between the first surface 226 and the second surface 228. The first surface 226, the second surface 228, and the thickness 222 may be similar to and operate as described above with respect to the first surface 126, the second surface 128, and the thickness 122.

Each aperture 224 may form a third surface 230 extending from the first surface 226 to the second surface 228 and forming a first edge 232, a second edge 236, a first diameter 234, and a second diameter 238. The third surface 230, the first edge 232, the first diameter 234, the second edge 236, and the second diameter 238 may be similar to and operate as described above with respect to the third surface 130, the first edge 132, the first diameter 134, the second edge 136, and the second diameter 138. In some embodiments, the third surface 230 may be a convex surface. For example, the third surface 230 may curve from the first edge 232 to the second edge 236. The third surface 230 may have a curve defined by a radius 252. In some embodiments, the radius 252 may be about the thickness 222 of the second adhesive layer 220 up to about three times the thickness 222 of the second adhesive layer 220.

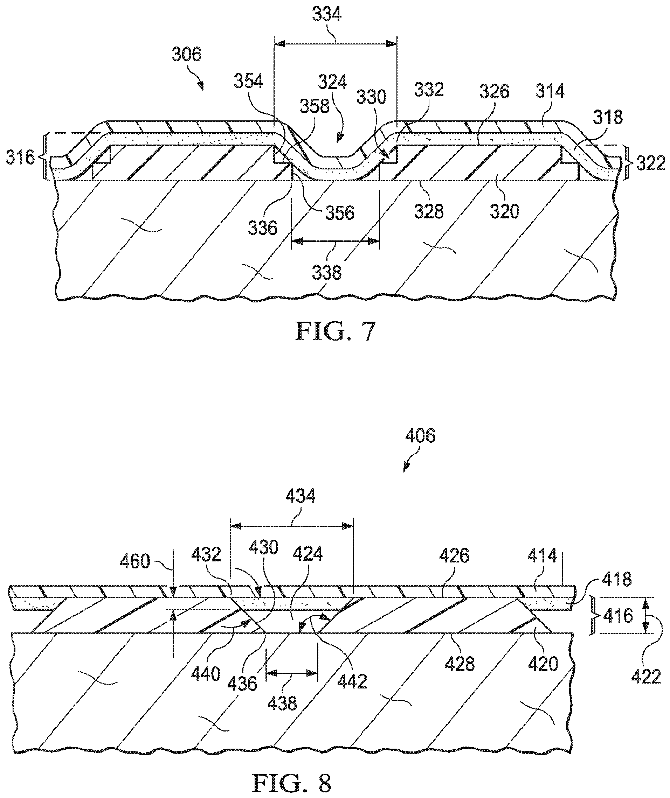

Figure 7 is a sectional view of a portion of another cover 306 illustrating additional details that may be associated with some embodiments. The cover 306 may include an elastomeric film 314 and an attachment device 316. The elastomeric film 314 and the attachment device 316 may be similar to and operate as described above with respect to the elastomeric film 114 and the attachment device 116. The attachment device 316 may include a first adhesive layer 318 and a second adhesive layer 320. The first adhesive layer 318 and the second adhesive layer 320 may be similar to and operate as described above with respect to the first adhesive layer 118 and the second adhesive layer 120. The second adhesive layer 320 may include a plurality of apertures 324 that may be similar to and operate as described above with respect to the plurality of apertures 124. The second adhesive layer 320 may have a first surface 326, a second surface 328, and a thickness 322. The first surface 326, the second surface 328, and the thickness 322 may be similar to and operate as described above with respect to the first surface 126, the second surface 128, and the thickness 122.

Each aperture 324 may form a third surface 330 extending from the first surface 326 to the second surface 328. In some embodiments, the third surface 330 may be a compound surface having a first cylindrical portion 354 having the first diameter 334 and a second cylindrical portion 356 having the second diameter 338. The first cylindrical portion 354 may extend from the first surface 326 toward the second surface 328 and may have a depth that is less than the thickness 322 of the second adhesive layer 320. In some embodiments, the first cylindrical portion 354 may have a depth that is about one-half the thickness 322. The second cylindrical portion 356 may extend from the second surface 328 toward the first surface 326 and may have a depth that is less than the thickness 322 of the second adhesive layer 320. In some embodiments, the second cylindrical portion 356 may have a depth that is about one-half the thickness 322. The first cylindrical portion 354 and the second cylindrical portion 356 may form a step 358 between the first surface 326 and the second surface 328. In some embodiments, the step 358 may be parallel to one or more of the first surface 326 and the second surface 328. If the step 358 is parallel to the first surface 326 and the second surface 328, a sum of the depths of the first cylindrical portion 354 and the second cylindrical portion 356 may be equal to the thickness 322. In other embodiments, the step 358 may form an angle with one or more of the first surface 326 and the second surface 328. If the step 358 forms an angle with the first surface 326 and the second surface 328, the sum of the depths of the first cylindrical portion 354 and the second cylindrical portion 356 may not be equal to the thickness 322.

Figure 8 is a sectional view of a portion of another cover 406 illustrating additional details that may be associated with some embodiments. The cover 406 may include an elastomeric film 414 and an attachment device 416. The elastomeric film 414 and the attachment device 416 may be similar to and operate as described above with respect to the elastomeric film 114 and the attachment device 116. The attachment device 416 may include a first adhesive layer 418 and a second adhesive layer 420. The first adhesive layer 418 and the second adhesive layer 420 may be similar to and operate as described above with respect to the first adhesive layer 118 and the second adhesive layer 120. The second adhesive layer 420 may include a plurality of apertures 424 that may be similar to and operate as described above with respect to the plurality of apertures 124. The second adhesive layer 420 may have a first surface 426, a second surface 428, and a thickness 422. The first surface 426. the second surface 428, and the thickness 422 may be similar to and operate as described above with respect to the first surface 126, the second surface 128, and the thickness 122. Each aperture 424 may form a third surface 430 extending from the first surface 426 to the second surface 428.

In other embodiments, the first adhesive layer 418 may be disposed on the elastomeric film 414 in a first pattern. The second adhesive layer 420 may be disposed directly on the elastomeric film 414 in a second pattern. The second pattern of the second adhesive layer 420 may be registered with the first pattern of the first adhesive layer 418. Registration of the first adhesive layer 418 and the second adhesive layer 420 generally refers to the alignment of the two adhesives relative to one another. In particular, registration of the first adhesive layer 418 and the second adhesive layer 420 may refer to the coordination of adhesive placement on the elastomeric film 414 to achieve a desired effect. For example, a certain percentage of overlap of one adhesive over the other adhesive, minimal overlap of one adhesive over the other adhesive so that the adhesives are offset from one another, or complete overlap of one adhesive over the other adhesive are all adhesive placements that may be considered registered. For example, the first adhesive layer 418 and the second adhesive layer 420 may be registered by being disposed on the elastomeric film 414 so that the first adhesive layer 418 and the second adhesive layer 420 each substantially couple to the elastomeric film 414. In addition, the first adhesive layer 418 and the second adhesive layer 420 may be aligned relative to one another to have minimal overlap of one adhesive over the other adhesive. In another example, the second adhesive layer 420 may be offset from the first adhesive layer 418, with both adhesives being coupled to the elastomeric film 414.

The second adhesive layer 420 is formed with a plurality of apertures 424. The plurality of apertures 424 may be similar to and operate as described above with respect to the plurality of apertures 124 and may include a third surface 430, a first edge 432, a first diameter 434, a second edge 436, a second diameter 438, a first angle 440, and a second angle 442. The first adhesive layer 418 may partially fill the plurality of apertures 424. For example, the first adhesive layer 418 may have a thickness 460. The thickness 460 may be less than the thickness 422 of the second adhesive layer 420. In some embodiments, the thickness 460 may be about one-half of the thickness 422.

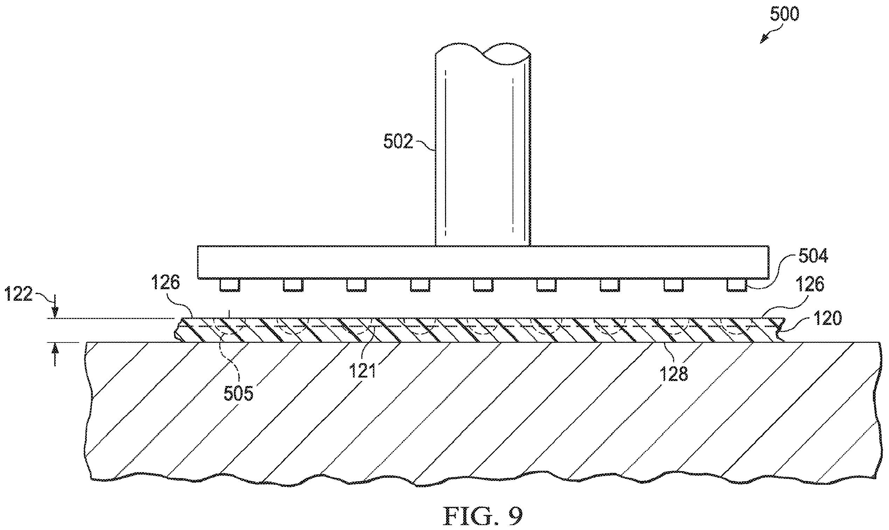

Figure 9 is a schematic view of a portion of a system 500 to form the apertures 124, illustrating additional details that may be associated with some embodiments. In the system 500, the second adhesive layer 120 may be provided in a cured state, such as cured roll-stock. In the cured state, the adhesive of the second adhesive layer 120 may be suitable for coupling to the first adhesive layer 118 and to epidermis adjacent to a tissue site. An adhesive in a cured state may have a penetration value between about 20 mm and about 22 mm under the International Standard for Organization cone penetration test ISO 2137 using a 62.5g mass and a 60 second contact time. The cured state may be contrasted with an un-cured or pre-cursor state. In the pre-cursor state, the adhesive of the second adhesive layer 120 may be in a solution form or not yet suitable for coupling to the first adhesive layer 118 or to epidermis adjacent to a tissue site. An adhesive in a pre-cursor state may have a penetration value much greater than 50 mm under the International Standard for Organization cone penetration test ISO 2137 using a 62.5g mass and a 60 second contact time. In some embodiments, the second adhesive layer 120 may have a scrim layer 121 disposed in the second adhesive layer 120. The scrim layer 121 may support the second adhesive layer 120 and aid in manipulation of the second adhesive layer 120.

The second adhesive layer 120 may be disposed on a surface, and a first tool 502 may be brought proximate to the first surface 126 of the second adhesive layer 120. The first tool 502 may include a plurality of heating elements 504. The heating elements 504 may be arranged on the first tool 502 so that a pitch between adjacent heating elements 504 corresponds with the pitch 144 and the pitch 146 of the apertures 124 of the second adhesive layer 120. The heating elements 504 may form heated portions 505 of the second adhesive layer 120. The heated portions 505 of the second adhesive layer 120 may be selected regions of the second adhesive layer 120 that have transitioned from the cured state to the pre-cursor state in response to heating by the heating elements 504 of the first tool 502. The heating elements 504 may heat the second adhesive layer 120 to form the heated portions 505 between about 1 second and about 60 seconds at a temperature between about 80 degrees Celsius and about 150 degrees Celsius. In other embodiments, the heating elements 504 may be another transitioning source, such as a plurality of ultraviolet light sources. The first adhesive layer 120 may be exposed to an ultraviolet light source having an intensity between about 100 mW/cm² and about 500 mW/cm² for between about 1 second and about 40 seconds to form the heated portions 505. After transitioning portions of the second adhesive layer 120 from the cured state to the pre-cursor state, the first tool 502 may be removed from the position proximate to the first surface 126 of the second adhesive layer 120.

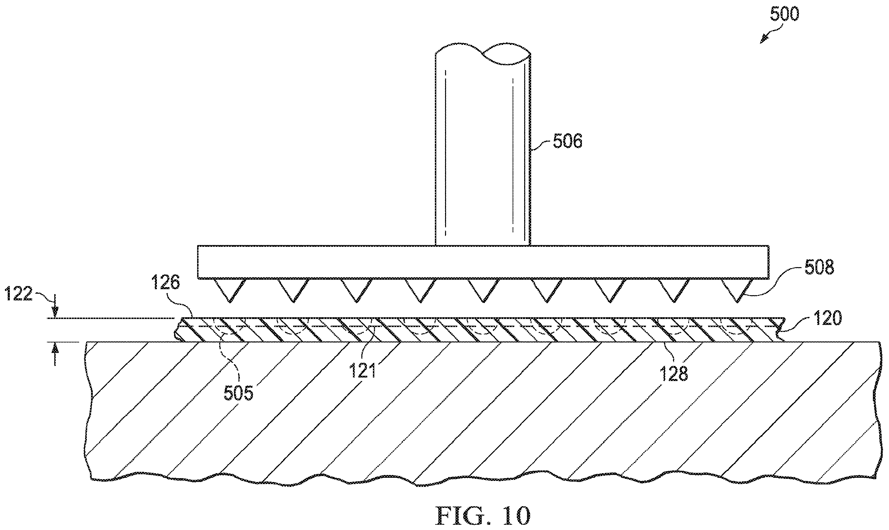

Figure 10 is a schematic view of a portion of the system 500 to form the apertures 124, illustrating additional details that may be associated with some embodiments. A second tool 506 may be brought proximate to the first surface 126 of the second adhesive layer 120. The second tool 506 may include a plurality of protrusions 508. In some embodiments, the plurality of protrusions 508 are conical. A base of each protrusion 508 may be coupled to the second tool 506 and extend away from the second tool 506 to an apex. Each of the protrusions 508 may have a base diameter approximately equal to the first diameter 134 of the apertures 124 of the second adhesive layer 120. A height of each protrusion 508 may be approximately equal to the thickness 122 of the second adhesive layer 120. In other embodiments, the height of each protrusion 508 may be greater than the thickness 122 of the second adhesive layer 120. The plurality of protrusions 508 may have a pitch between adjacent protrusions 508 that corresponds to the pitch 144 and the pitch 146 between adjacent apertures 124 of the second adhesive layer 120. As the second tool 506 is brought proximate to the first surface 126 of the second adhesive layer 120, the protrusions 508 may be aligned with the heated portions 505 formed by the first tool 502. In other embodiments, the plurality of protrusions 508 may have other shapes, for example spherical, cuboid, or amorphous shapes.

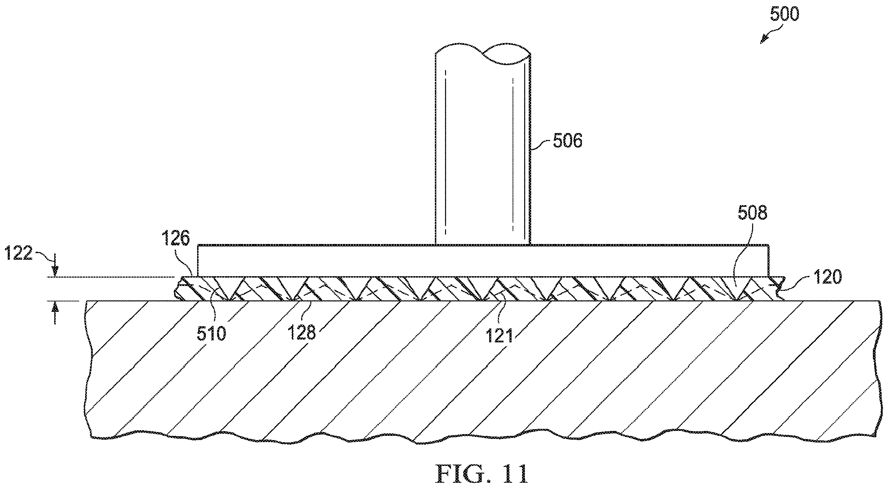

Figure 11 is a schematic view of a portion of the system 500 to form the apertures 124, illustrating additional details that may be associated with some embodiments. The second tool 506 may be pressed against the first surface 126 of the second adhesive layer 120. Pressing the second tool 506 against the first surface 126 of the second adhesive layer 120, presses the protrusions 508 into the second adhesive layer 120. In some embodiments, the protrusions 508 are registered with the heated portions 505 of the second adhesive layer 120. In the precursor state, the heated portions 505 may be flowable. As the protrusions 508 are pressed into the second adhesive layer 120, the protrusions 508 can displace the adhesive forming the second adhesive layer 120. Displacement of the adhesive forming the second adhesive layer 120 can form depressions 510 in the second adhesive layer 120. The depressions 510 may have a shape matching the shape of the protrusions 508.

If the protrusions 508 have a height greater than the thickness 122 of the second adhesive layer 120, a surface of the second tool 506 proximate to the first surface 126 of the second adhesive layer 120 may be spaced from the first surface 126 of the second adhesive layer 120. As the protrusions 508 penetrate the second adhesive layer 120 at the heated portions 505, the adhesive may flow into areas surrounding the heated portions 505. In some embodiments, the flow of the adhesive into areas surrounding the heated portions 505 may increase the coating weight of the second adhesive layer 120. For example, if the second adhesive layer 120 is provided to the system 500 having a first coating weight of about 150 gsm, the displacement of the adhesive forming the second adhesive layer 120 by the protrusions 508 may increase the coating weight in areas surrounding the heated portions 505 to about 250 gsm. In some embodiments, the thickness 122 of the second adhesive layer may increase from about 150 microns to about 250 microns. In some embodiments, the protrusions 508 may deform the scrim layer 121 so that the scrim layer 121 does not cross the third surface 130.

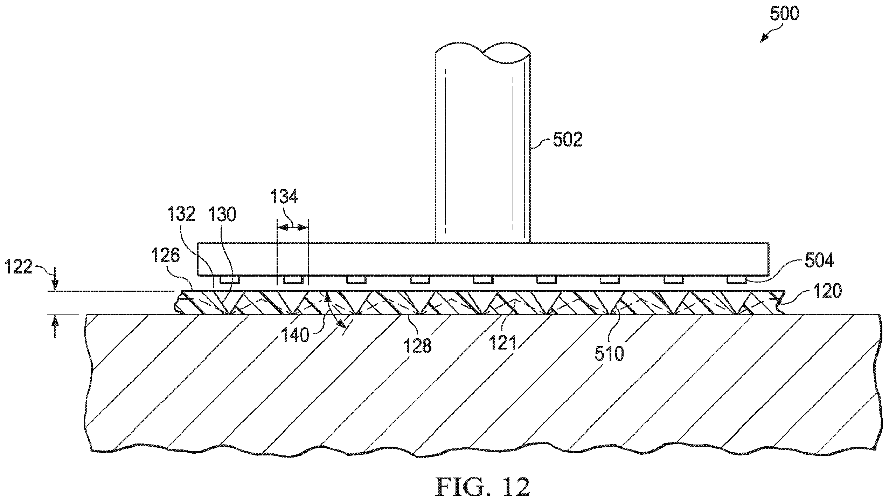

Figure 12 is a schematic view of a portion of the system 500 to form the apertures 124, illustrating additional details that may be associated with some embodiments. The second tool 506 may be removed, and the first tool 502 may be brought proximate to the second adhesive layer 120. The heating elements 504 may heat the depressions 510 of the second adhesive layer 120, transitioning the depressions 510 of the second adhesive layer 120 from the pre-cursor state to the cured state.

In other embodiments, the second tool 506 may be configured to apply heat to the second adhesive layer 120. For example, the second tool 506 may be capable of heating the second adhesive layer 120 to form the heated portions 505, transitioning the heated portions from the cured to the pre-cursor state. The second tool 506 may also be capable of displacing the adhesive of the second adhesive layer 120 to form the depressions 510. The second tool 506 may also be capable of re-applying heat to the depressions 510 to transition the heated portions 505 and the depressions 510 from the pre-cursor state to the cured state.

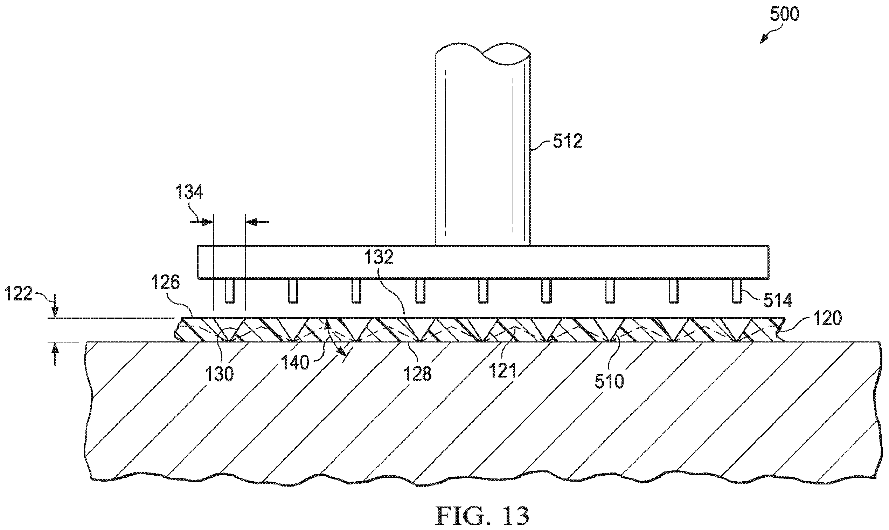

Figure 13 is a schematic view of a portion of the system 500 to form the apertures 124, illustrating additional details that may be associated with some embodiments. After transitioning the depressions 510 from the pre-cursor state to the cured state, the first tool 502 may be removed and a third tool 512 may be brought proximate to the second adhesive layer 120. The third tool 512 may be a cutting tool or a registered clicker press tool having a plurality of scalpets 514. The scalpets 514 may have a pitch between adjacent scalpets 514 that is approximately equal to the pitch 144 and the pitch 146 between adjacent apertures 124 of the second adhesive layer 120. The scalpets 514 may be positioned on the third tool 512 so that, if the third tool 512 is brought proximate to the second adhesive layer 120, the scalpets 514 may be aligned with, i.e. registered, with the depressions 510. Preferably, a center of each scalpet 514 is aligned with a center of each depression 510. In some embodiments, the scalpets 514 may have a length greater than or equal to the thickness 122 of the second adhesive layer 120. The scalpets 514 may have a cutting diameter equal to the second diameter 138.

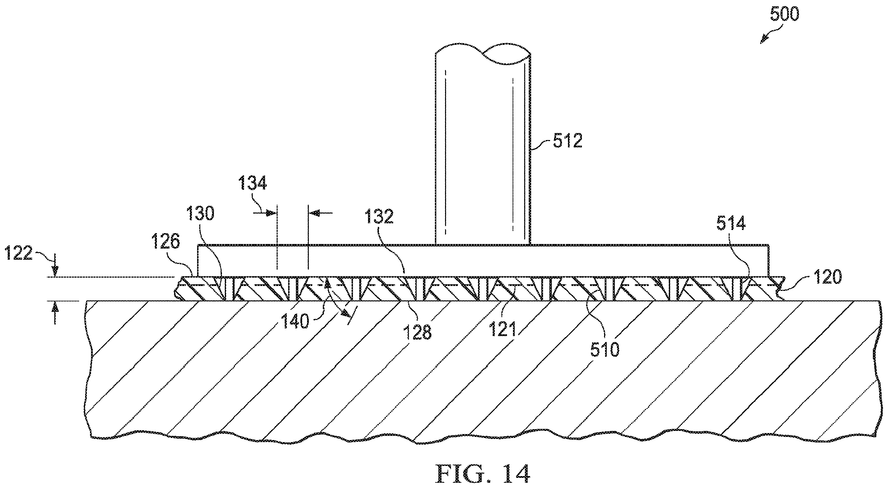

Figure 14 is a schematic view of a portion of the system 500 to form the apertures 124, illustrating additional details that may be associated with some embodiments. The third tool 512 may be pressed against the first surface 126 of the second adhesive layer 120. If the third tool 512 is pressed against the first surface 126, the scalpets 514 maybe inserted into and through the depressions 510. Insertion of the scalpets 514 into the depressions 510 may cut the depressions 510. In some embodiments, the scalpets 514 may cut through the scrim layer 121, leaving the apertures 124 free from the scrim layer 121.

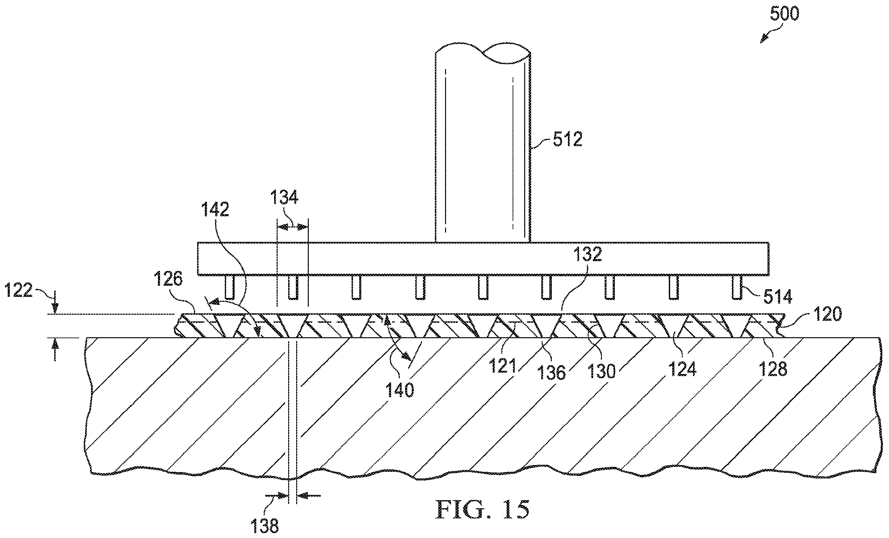

Figure 15 is a schematic view of a portion of the system 500 to form the apertures 124, illustrating additional details that may be associated with some embodiments. The third tool 512 may be moved out of contact with the first surface 126. As the third tool 512 is moved away from the second adhesive layer 120, portions of the depressions 510, cut by the scalpets 514, may be removed from the second adhesive layer 120. Removal of portions of the depressions 510 can form the apertures 124 having the second diameter 138 in the second surface 128. Following formation of the apertures 124, the first adhesive layer 118 and the elastomeric film 114 may be coupled to the second adhesive layer 120 to form the cover 106. For example, the first adhesive layer 118 and the elastomeric film 114 can be coupled to the first surface 126 of the second adhesive layer 120. In some embodiments, one or more release liners may be releasably coupled to surfaces of the cover 106. For example, a release liner may be coupled to the second surface 128 of the second adhesive layer 120.

As described in Figures 9-15, the apertures 124 are formed by using local heat and compression to deform portions of the second adhesive layer 120. The material displaced by the protrusions 508 to form the depressions 510 may flow into adjacent areas of the second adhesive layer 120. In some embodiments, flow of the material out of the depressions 510 and into surrounding areas of the second adhesive layer 120 may increase a coating weight of the adhesive forming the second adhesive layer 120. For example, the second adhesive layer 120 may be provided having a coating weight of about 150 gsm. Following heating by the first tool 502 having the plurality of heating elements 504 and compression by the second tool 506 having the plurality of protrusions 508, the adhesive of the second adhesive layer 120 may have a coating weight of about 250 gsm. The increase in the coating weight of the adhesive of the second adhesive layer 120 can be caused by the flow of the adhesive in the pre-cursor state out of the depressions 510 and into the remainder of the second adhesive layer 120. In some embodiments, the second tool 506 may also be capable of heating the second adhesive layer 120. Heating the second adhesive layer 120 may encourage flow of the adhesive forming the second adhesive layer 120.

Displacement of the adhesive forming the second adhesive layer 120 out of the depressions 510 can permit more efficient use of the adhesive. For example, increasing the coating weight of the second adhesive layer 120 by displacing adhesive out of the depressions 510 can decrease the amount of material needed to form the cover 106 by using up to 30% less adhesive to manufacture the cover 106. In some embodiments, the adhesive forming the second adhesive layer 120 may be cured after formation of the depressions 510 or be capable of a delayed cure that permits heating by the first tool 502 to fix the form of the depressions 510.

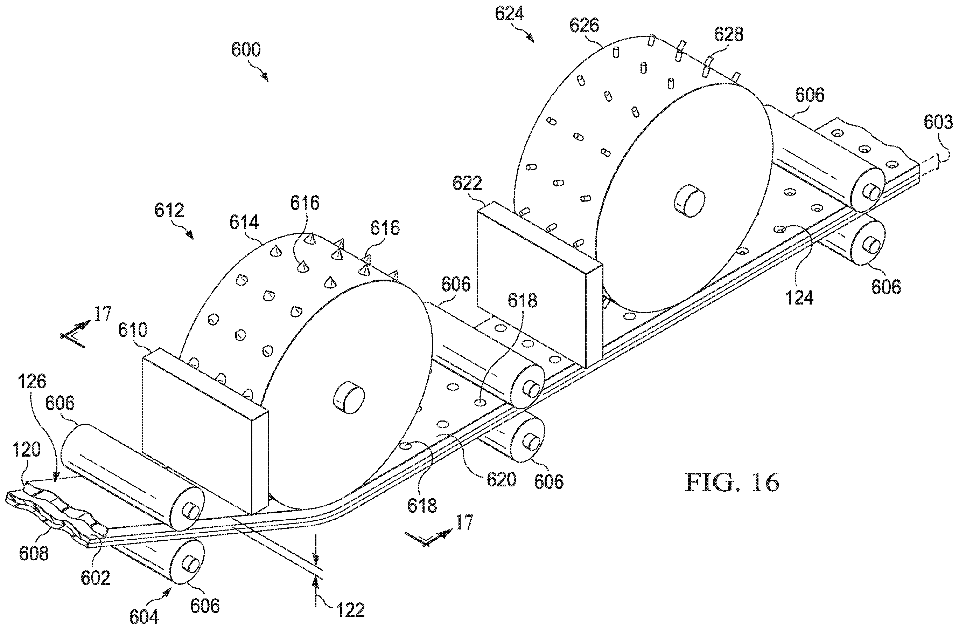

Figure 16 is a schematic perspective view of a system 600 for forming the apertures 124 in the second adhesive layer 120 illustrating additional details that may be associated with some embodiments. The second adhesive layer 120 may be supplied to the system 600 linearly after being coated onto a carrier material. For example, the adhesive forming the second adhesive layer 120 may be coated onto a carrier layer 602 and cured from the pre-cursor state to the cured state to form a drape assembly 603. The carrier layer 602 may be a release liner configured to be releasably coupled to the second adhesive layer 120. The carrier layer 602 may be one or more of the following: a polyurethane film, a high density polyethylene, a high-MVTR film, polymers such as acrylic copolymers, polyvinyl acetate, polyether block amide copolymers (PEBAX), polyvinyl alcohol and copolymers, polyamide, polyvinylchloride, or polyvinylidene chloride. In some embodiments, the adhesive forming the second adhesive layer 120 may be provided to the system 600 in the pre-cursor state or in a partially-cured state.

The drape assembly 603 may be provided on a conveyor assembly 604 having a belt 608. The conveyor assembly 604 may be, for example, a belt conveyor, gravity conveyor, bucket conveyor, roller conveyor, chain conveyor, vibrating conveyor, or other suitable device, configured to transport the drape assembly 603 through the system 600. The conveyor assembly 604 may be one or more conveyor systems or a single conveyor system as schematically illustrated in Figure 16. The conveyor assembly 604 may include additional components not illustrated or described herein that can support and operate the described components. The conveyor assembly 604 may include one or more rollers 606. The rollers 606 may be configured to cause translation of a belt or carrier, such as a belt 608, and may be free-spinning or motorized. In some embodiments, the drape assembly 603 may be provided to the conveyor assembly 604 in sheets that may appear continuous to the conveyor assembly 604. For example, the drape assembly 603 may be provided in rolls that may be disposed onto the conveyor assembly 604. The conveyor assembly 604 may unroll the roll of the drape assembly 603 as the conveyor assembly 604 moves a first end of the drape assembly 603 through the process. The drape assembly 603 may be disposed on the conveyor assembly 604 so that the first surface 126 of the second adhesive layer 120 may face away from the belt 608 of the conveyor assembly 604. In some embodiments, the drape assembly 603 may move in response to movement of the belt 608 of the conveyor assembly 604.

As shown in Figure 16, the conveyor assembly 604 may convey the drape assembly 603 and the second adhesive layer 120 to a first cross-linking apparatus 610. The first cross-linking apparatus 610 may be a heat source or an ultraviolet light source configured to cross-link the adhesive of the second adhesive layer 120. Preferably, the first cross-linking apparatus 610 can transition the adhesive of the second adhesive layer 120 to the pre-cursor state. For example, if the adhesive of the second adhesive layer 120 is provided in a cured or a partially-cured state, the first cross-linking apparatus 610 can apply heat or ultraviolet light to crosslink the adhesive, causing the adhesive to transition to the pre-cursor state. The first cross-linking apparatus 610 can be sized so that the first cross-linking apparatus 610 can transition the entirety of the second adhesive layer 120 as it passes through the first cross-linking apparatus 610. The second adhesive layer 120 may be heated between about 1 second and about 60 seconds at a temperature between about 80 degrees Celsius and about 150 degrees Celsius. In other embodiments, the first adhesive layer 120 may be exposed to an ultraviolet light source having an intensity between about 100 mW/cm² and about 500 mW/cm² for between about 1 second and about 40 seconds.

The conveyor assembly 604 can convey the carrier layer 602 and the second adhesive layer 120 to a first compression assembly, such as a compression assembly 612. The compression assembly 612 may include additional components not illustrated herein that support and operate the components described below. The compression assembly 612 may include a first compression roller, such as a compression roller 614. The compression roller 614 may be a cylinder having a side surface. The compression roller 614 may have one or more protrusions 616 protruding from the side surface of the compression roller 614. The protrusions 616 may be arranged on the compression roller 614 so that a pitch between adjacent protrusions 616 corresponds with the pitch 144 and the pitch 146 of the apertures 124 of the second adhesive layer 120. The protrusions 616 may be conical. In some embodiments, each protrusion 616 may have a base coupled to the side surface of the compression roller 614. The base of each protrusion 616 may have a diameter that is about equal to the first diameter 134 of the apertures 124 of the second adhesive layer 120. The protrusions 616 may extend outwardly from the side surface of the compression roller 614 to an apex. The protrusions 616 may have a height from the base to the apex that is greater than the thickness 122 of the second adhesive layer 120. In other embodiments, the protrusions 616 may be other shapes, for example spherical, cuboid, stepped, amorphous, or may form convex surfaces.

As the carrier layer 602 and the second adhesive layer 120 are conveyed through the compression assembly 612, the compression roller 614 presses the protrusions 616 into the first surface 126 of the second adhesive layer 120, displacing the adhesive forming the second adhesive layer 120 to form a plurality of depressions 618. The side surface of the compression roller 614 may encourage the adhesive displaced by the protrusions 616 to flow into adjacent areas 620 of the second adhesive layer 120. In some embodiments, the material displaced by the protrusions 616 to form the depressions 618 may flow into the adjacent areas 620 of the second adhesive layer 120.

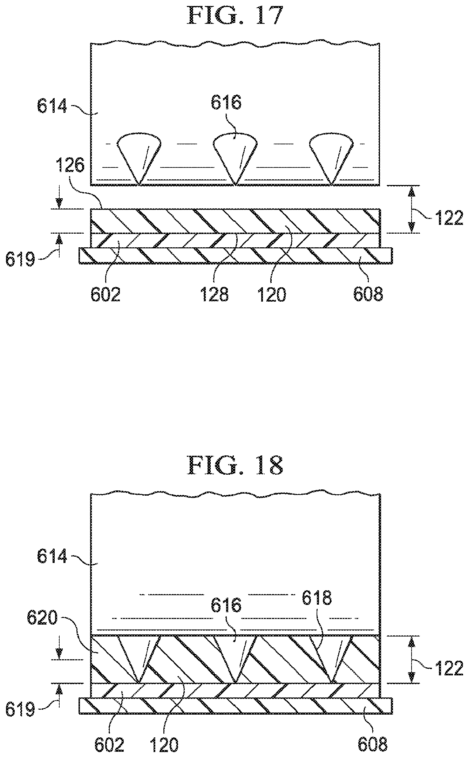

Figure 17 is a sectional schematic view of a portion of the system 600 taken along line 17-17 of Figure 16 illustrating additional details that may be associated with some embodiments. As shown in Figure 17, the second adhesive layer 120 may have a thickness 619. The thickness 619 of the second adhesive layer 120 may be less than the thickness 122 of the second adhesive layer 120 following conclusion of the manufacturing process. In some embodiments, the side surface of the compression roller 614 may be spaced from the first surface 126 of the second adhesive layer 120. For example, the compression roller 614 may be positioned so that the side surface of the compression roller 614 is a distance from the surface of the carrier layer 602 that is about equal to the thickness 122.

Figure 18 is a sectional schematic view of a portion of the system 600, illustrating additional details that may be associated with some embodiments. As the protrusions 616 press into the adhesive layer 120, the adhesive is displaced. The displacement of the adhesive forms the depressions 618 and can increase the thickness of the adhesive layer 120 from the thickness 619 to the thickness 122. Flow of the material out of the depressions 618 and into the adjacent areas 620 of the second adhesive layer 120 may increase a coating weight of the adhesive forming the second adhesive layer 120. For example, the second adhesive layer 120 may be provided on the carrier layer 602 having a coating weight of about 150 gsm. Following compression by the compression roller 614, the adhesive of the second adhesive layer 120 may have a coating weight of about 250 gsm. The increase in the coating weight of the adhesive of the second adhesive layer 120 can be caused by the flow of the adhesive in the pre-cursor state out of the depressions 618 and into the adjacent areas 620 of the second adhesive layer 120. The side surface of the compression roller 614 may encourage the displaced adhesive to flow uniformly across the second adhesive layer 120. For example, the compression roller 614 may be positioned a predetermined distance from the first surface 126 of the second adhesive layer 120. As the adhesive flows from the depressions 618 to the other areas 620, the adhesive may be constrained from building vertically beyond the thickness 122, causing the adhesive to flow parallel to the side surface of the compression roller 614.

Displacement of the adhesive forming the second adhesive layer 120 out of the depressions 618 can permit more efficient use of the adhesive. For example, increasing the coating weight of the second adhesive layer 120 by displacing adhesive out of the depressions 618 uniformly across the second adhesive layer 120 can increase the efficiency of the process of manufacturing the cover 106 by saving up to 30% of the material needed to manufacture the cover 106.

Referring to Figure 16, the conveyor assembly 604 may continue to move the drape assembly 603 following passage of the carrier layer 602 and the second adhesive layer 120 through the compression assembly 612. The conveyor assembly 604 may move the drape assembly 603 past a curing or drying assembly, such as a second cross-linking assembly 622. The second cross-linking assembly 622 may be a suitable apparatus configured to cure, dry, or crosslink the second adhesive layer 120 while the conveyor assembly 604 moves the carrier layer 602 and the second adhesive layer 120 past the second cross-linking assembly 622. The second cross-linking assembly 622 can fix the second adhesive layer 120 so that the adhesive of the second adhesive layer 120 does not flow into and fill the depressions 618. Fixing the second adhesive layer 120 can refer to transitioning the adhesive of the second adhesive layer 120 from the pre-cursor state to the cured state. Following the fix of the second adhesive layer 120 having the depressions 618, the drape assembly 603 may be stored as roll-stock or moved to another manufacturing assembly for further processes.

In some embodiments, the conveyor assembly 604 may convey the drape assembly 603 to a second compression assembly, such as a compression assembly 624. The compression assembly 624 may include additional components not illustrated herein that support and operate the components described below. The compression assembly 624 may include a second compression roller or rotary cutting tool, such as compression roller 626. The compression roller 626 may be a cylinder having a side surface. The compression roller 626 may have a plurality of scalpets 628 extending from the side surface of the compression roller 626. The scalpets 628 may be arranged on the compression roller 626 so that a pitch between adjacent scalpets 628 corresponds with the pitch 144 and the pitch 146 of the apertures 124 of the second adhesive layer 120. The scalpets 628 may be positioned on the compression roller 626 so that, as the carrier layer 602 and the second adhesive layer 120 pass through the compression assembly 624, the scalpets 628 may be aligned with, i.e. registered, with the depressions 618. Preferably, a center of each scalpet 628 is aligned with a center of each depression 618. In some embodiments, the scalpets 628 may have a length greater than or equal to the thickness 122 of the second adhesive layer 120. The scalpets 628 may have a cutting diameter equal to the second diameter 138. As the second adhesive layer 120 moves through the compression assembly 624, the scalpets 628 may cut a portion of each depression 618 from each depression 618, forming the apertures 124.

In some embodiments, the carrier layer 602 and the second adhesive layer 120 may be conveyed through additional assemblies that can couple the first adhesive layer 118, the elastomeric film 114, and any desired release liners to the second adhesive layer 120 to form the cover 106. For example, the first adhesive layer 118 can be coupled to the elastomeric film 114. The first adhesive layer 118 and the elastomeric film 114 can then be coupled to the second adhesive layer 120 to form the cover 106. In some embodiments, the carrier layer 602 may be used as a release liner for the cover 106.

In some embodiments, the compression assembly 612 and the compression assembly 624 may be combined. For example, the protrusions 616 and the scalpets 628 may be combined as protruding scalpets on a compression assembly. The protruding scalpets may be conical and have a base diameter coupled to a compression roller that is about equal to the first diameter 134 of the apertures 124 of the second adhesive layer 120. The protruding scalpets may extend outwardly from the side surface of the compression roller to a distal end. The protruding scalpets may have a height from the base to the distal end that is greater than the thickness 122 of the second adhesive layer 120. The distal end of each protruding scalpet may have a cutting diameter equal to the second diameter 138. As the second adhesive layer 120 moves through the combined compression assembly, the protruding scalpets may displace adhesive and cut adhesive forming both depressions and the apertures.

The systems, apparatuses, and methods described herein may provide significant advantages. For example, the covers described herein improve the adhesion area used to adhere the cover to a patient without reducing the sealing area sealed by the second adhesive layer. By forming a chamfer in each aperture, more of the first adhesive layer can contact tissue of the tissue site, increasing the adhesion area of the cover. In some embodiments, the covers described herein may have an adhesion area that is 20% greater than the adhesion area of other similar covers. The covers described herein also make more efficient use of the adhesives during the manufacturing process. For example, by displacing the adhesive of the second adhesive layer to form the apertures, a thinner initial layer of adhesive can be used. Furthermore, by displacing the adhesive of the second adhesive layer, less adhesive is removed from the second adhesive while forming the apertures, decreasing waste of adhesive. Because the first adhesive layer fills the apertures in the second adhesive layer, the covers described herein also do not need the application of an external force, such as hand pressure, to place the first adhesive layer into contact with epidermis of a patient. In some embodiments, the first adhesive layer is closer to the epidermis upon application of the cover over other hybrid covers. This permits the cover to be applied without requiring hand pressure to force the adhesive through the holes. The covers can also still be repositioned. The covers described herein can also be less costly to produce over other similar covers as a lower initial coat-weight may be used to achieve a similar maximum thickness when compared to other similar covers. Furthermore, the covers described herein can have a more flexible edge to the second adhesive layer, permitting the second adhesive layer to be more likely to flow into small crevices and creases in tissue and provide a better seal over other similar covers.

While shown in a few illustrative embodiments, a person having ordinary skill in the art will recognize that the systems, apparatuses, and methods described herein are susceptible to various changes and modifications. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the dressing, the container, or both may be eliminated or separated from other components for manufacture or sale. In other example configurations, a controller may also be manufactured, configured, assembled, or sold independently of other components.

The appended claims set forth novel and inventive aspects of the subject matter described above, but the claims may also encompass additional subject matter not specifically recited in detail. For example, certain features, elements, or aspects may be omitted from the claims if not necessary to distinguish the novel and inventive features from what is already known to a person having ordinary skill in the art. Features, elements, and aspects described herein may also be combined or replaced by alternative features serving the same, equivalent, or similar purpose without departing from the scope of the invention defined by the appended claims.

Aspects of the invention are disclosed in the following numbered clauses:
1. A sealing member for providing a seal over a tissue site, the sealing member comprising:
   a film layer;
   a first adhesive coupled to and coextensive with the film layer;
   a second adhesive coupled to and coextensive with the first adhesive;
   a plurality of apertures extending through the second adhesive, each aperture
      defining an interior surface between a first side and a second side of the second adhesive, the interior surface intersecting the first side to form a first edge and intersecting the second side to form a second edge, the interior surface comprising a chamfer; and
   wherein the first adhesive is configured to extend at least partially through the plurality of apertures.
2. The sealing member of claim 1, wherein the chamfer is about 30 degrees.
3. The sealing member of claim 1, wherein the chamfer is about 45 degrees.
4. The sealing member of claim 1, wherein the chamfer is between about 20 degrees and about 60 degrees.
5. The sealing member of claim 1, wherein the second edge has a diameter of about 10 mm.
6. The sealing member of claim 1, wherein the second adhesive has a coating weight of about 250 gsm.
7. A cover for providing a seal over a tissue site, the cover comprising:
   a polyurethane layer;
   an acrylic adhesive coupled to and coextensive with the polyurethane layer;
   a silicone adhesive coupled to and coextensive with the acrylic adhesive; a plurality of openings extending through the silicone adhesive, each opening having a first diameter opposite the acrylic adhesive and a second diameter adjacent the acrylic adhesive; and
   wherein the acrylic adhesive is configured to extend at least partially through the plurality of openings.
8. The cover of claim 7, wherein the second diameter is larger than the first diameter.
9. The cover of claim 7, wherein a surface between the first diameter of each opening and the second diameter of each opening is curved.
10. The cover of claim 9, wherein the surface is a convex curve.
11. The cover of claim 7, wherein a surface between the first diameter of each opening and the second diameter of each opening forms a chamfer.
12. The cover of claim 11, wherein the chamfer is about 45 degrees.
13. The cover of claim 11, wherein the chamfer is between about 20 degrees and about 60 degrees.
14. The cover of claim 7, wherein the first diameter is about 10 mm.
15. The cover of claim 7, wherein the second diameter is about 11 mm.
16. The cover of claim 7, wherein the silicone adhesive has a coating weight of about 250 gsm.
17. A drape for providing a seal over a tissue site, the drape comprising:
   a sealing layer having a plurality of apertures extending through the sealing layer; a plurality of tapered surfaces formed by the plurality of apertures in the sealing layer;
   a bonding layer coupled to the sealing layer; and
   a barrier layer coupled to the bonding layer on a side of the bonding layer opposite the sealing layer.
18. The drape of claim 17, wherein an angle formed by each tapered surface of the plurality of tapered surfaces with a side of the sealing layer is about 45 degrees.
19. The drape of claim 17, wherein an angle formed by each tapered surface of the plurality of tapered surfaces with a side of the sealing layer is between about 20 degrees and about 60 degrees.
20. The drape of claim 17, wherein the plurality of tapered surfaces form a first opening having a first diameter on a first side of the sealing layer and a second opening having a second diameter on a second side of the sealing layer.
21. The drape of claim 20, wherein the second side of the sealing layer is adjacent to the bonding layer.
22. The drape of claim 20, wherein the first diameter is about 10 mm.
23. The drape of claim 22, wherein the second diameter is about 11 mm.
24. The drape of claim 20, wherein the second diameter is about 11 mm.
25. The drape of claim 17, wherein the sealing layer has a coating weight of about 250 gsm.
26. A method of manufacturing a sealing member comprising:
   providing a carrier layer;
   coating the carrier layer with a first adhesive;
   curing the first adhesive to form a first adhesive layer;
   applying energy to a plurality of circular areas of the first adhesive layer to transition the circular areas from a cured state to a precursor state;
   compressing the first adhesive layer to form a plurality of depressions registered with the plurality of circular areas;
   applying energy to the plurality of circular areas of the first adhesive layer to
      transition the circular areas from the precursor state to the cured state;
   removing at least a portion of each depression of the first adhesive layer to form an aperture registered with each depression;
   coupling a second adhesive to a film layer to form a second adhesive layer;
   coupling the first adhesive layer to the second adhesive layer; and
   removing the carrier layer.
27. The method of claim 26, wherein applying energy to a plurality of circular areas of the first adhesive layer to transition the circular areas from a cured state to a precursor state comprises heating the first adhesive layer.
28. The method of claim 26, wherein applying energy to a plurality of circular areas of the first adhesive layer to transition the circular areas from a cured state to a precursor state comprises exposing the first adhesive layer to an ultra-violet light.
29. The method of claim 26, wherein applying energy to the plurality of circular areas of the first adhesive layer to transition the circular areas from the precursor state to the cured state comprises heating the first adhesive layer.
30. The method of claim 26, wherein applying energy to the plurality of circular areas of the first adhesive layer to transition the circular areas from the precursor state to the cured state comprises exposing the first adhesive layer to an ultra-violet light.
31. The method of claim 26, wherein each depression has a diameter on a surface of the first adhesive layer of about 11 mm.
32. The method of claim 26, wherein each aperture has a diameter smaller than a diameter of a corresponding depression.
33. The method of claim 26, wherein a diameter of each aperture on a surface of the first adhesive layer is about 10 mm.
34. The method of claim 26, wherein compressing the first adhesive layer to form a plurality of depressions registered with the plurality of circular areas comprises displacing the first adhesive from the circular areas of the first adhesive layer.
35. The method of claim 26, wherein compressing the first adhesive layer to form a plurality of depressions registered with the plurality of circular areas comprises:
   applying heat to the first adhesive layer; and
   inducing flow of the first adhesive to uniformly distribute the first adhesive displaced from the depressions across the first adhesive layer.
36. The method of claim 35, wherein the first adhesive has a first coating weight of about 150 gsm and a second coating weight of about 250 gsm after compressing the circular areas of the first adhesive layer to form depressions.
37. A method of manufacturing a cover comprising:
   supplying a first adhesive layer on carrier layer, the first adhesive layer comprising a silicone adhesive in a cured state;
   supplying energy to the first adhesive layer to transition the silicone adhesive from a cured state to a precursor state;
   forming a plurality of depressions in the silicone adhesive of the first adhesive layer; supplying energy to the first adhesive layer to transition the silicone adhesive from the precursor state to the cured state;
   removing a portion of the silicone adhesive at each depression of the first adhesive layer to form a plurality of openings registered with the plurality of depressions;
   coupling a second adhesive layer to a film layer;
   coupling the first adhesive layer to the second adhesive layer; and
   removing the carrier layer.
38. The method of claim 37, wherein supplying energy to the first adhesive layer to transition the silicone adhesive from the cured state to the precursor state comprises heating the first adhesive layer.
39. The method of claim 37, wherein supplying energy to the first adhesive layer to transition the silicone adhesive from the cured state to the precursor state comprises exposing the first adhesive layer to an ultra-violet light.
40. The method of claim 37, wherein forming a plurality of depressions in the silicone adhesive of the first adhesive layer comprises displacing the silicone adhesive into other areas of the first adhesive layer.
41. The method of claim 40, wherein displacing the silicone adhesive into other areas of the first adhesive layer comprises uniformly distributing the silicone adhesive displaced from the depressions across the first adhesive layer.
42. The method of claim 41, wherein the first adhesive layer has a first coating weight of about 150 gsm and a second coating weight of about 250 gsm, the first adhesive layer transitioning from the first coating weight to the second coating weight in response to displacing the silicone adhesive into other areas of the first adhesive layer.
43. The method of claim 37, wherein supplying energy to the first adhesive layer to transition the silicone adhesive from the precursor state to the cured state comprises heating the first adhesive layer.
44. The method of claim 37, wherein supplying energy to the first adhesive layer to transition the silicone adhesive from the precursor state to the cured state comprises exposing the first adhesive layer to an ultra-violet light.
45. The method of claim 37, wherein removing a portion of the silicone adhesive at each depression of the first adhesive layer comprises using a registered clicker press tool to form the opening in each depression of the first adhesive layer.
46. The method of claim 37, wherein removing a portion of the silicone adhesive at each depression of the first adhesive layer comprises using a rotary cutting tool to form the opening in each depression of the first adhesive layer.
47. The method of claim 37, wherein the depressions are conical depressions.
48. The method of claim 37, wherein the depressions are semi-spherical depressions.
49. The systems, methods, and apparatuses substantially as described herein.

## Claims

1. A drape for providing a seal over a tissue site, the drape comprising:
a sealing layer having a plurality of apertures extending through the sealing layer;
a plurality of tapered surfaces formed by the plurality of apertures in the sealing layer;
a bonding layer coupled to the sealing layer; and
a barrier layer coupled to the bonding layer on a side of the bonding layer opposite the sealing layer.

2. The drape of claim 1, wherein an angle formed by each tapered surface of the plurality of tapered surfaces with a side of the sealing layer is about 45 degrees.

3. The drape of claim 1, wherein an angle formed by each tapered surface of the plurality of tapered surfaces with a side of the sealing layer is between about 20 degrees and about 60 degrees.

4. The drape of claim 1, wherein the plurality of tapered surfaces form a first opening having a first diameter on a first side of the sealing layer and a second opening having a second diameter on a second side of the sealing layer.

5. The drape of claim 4, wherein the second side of the sealing layer is adjacent to the bonding layer.

6. The drape of claim 4, wherein the first diameter is about 10 mm.

7. The drape of claim 6, wherein the second diameter is about 11 mm.

8. The drape of claim 4, wherein the second diameter is about 11 mm.

9. The drape of claim 1, wherein the sealing layer has a coating weight of about 250 gsm.
